# EUROPEAN PATENT APPLICATION

(11) **EP 2 896 419 A1**
(43) Date of publication of application: **22.07.2015**
(21) Application number: 14187186.3
(22) Date of filing: 23.03.2006
(51) Int. Cl.: A61M 5/145, A61M 5/168

(54) **Fluid delivery device**

(30) Priority: 28.03.2005 US 907287; 28.03.2005 US 907286
(62) Divisional of application: 06739271.2
(71) Applicant: Insulet Corporation, Bedford, MA 01730 (US)
(72) Inventor: Dilanni, Steven, Danvers, MA Massachusetts 01923 (US); James, Brian, Lexinton, MA 02421 (US); Garibotto, John, Marblehead, MA 01945 (US); Zeller, David, Malden, MA 02148 (US); Daigle, Jason, Medford, MA 02155 (US); Sacco, Victor Jr, Haverhill, MA 01835 (US)
(74) Representative: Hill, Justin John

(57) **Abstract**

A fluid delivery device may include a fluid driving mechanism for a driving fluid out of a reservoir and an actuating mechanism for actuating the driving mechanism. The fluid delivery device may also include one or more sensors for monitoring operation of the fluid delivery device. The fluid delivery device may also include a chassis providing mechanical and/or electrical connections between components of the fluid delivery device.

## Description

### CROSS-REFERENCE TO RELATED APPLICATIONS

This application is a continuation-in-part of co-pending U.S. Patent Application Serial No. 10/704291, filed on November 7, 2003, which is a continuation-in-part of co-pending U.S. Patent Application Serial No. 10/128,205, which are assigned to the assignee of the present application and are incorporated herein by reference.

This application is related to U.S. Patent Application Serial No. 10/907286 entitled CHASSIS FOR FLUID DELIVERY DEVICE, which is filed concurrently herewith, assigned to the assignee of the present application, and incorporated herein by reference.

### TECHNICAL FIELD

The present invention relates to fluid delivery devices and more particularly, to an infusion pump for delivering therapeutic liquids to a patient.

### BACKGROUND INFORMATION

Fluid delivery devices have numerous uses such as delivering a liquid medicine to a patient subcutaneously. In a patient with diabetes mellitus, for example, ambulatory infusion pumps have been used to deliver insulin to a patient. These ambulatory infusion pumps have the ability to offer sophisticated fluid delivery profiles including variable basal rates and bolus requirements. The ability to carefully control drug delivery can result in better efficacy of the drug and therapy and less toxicity to the patient.

Some existing ambulatory infusion pumps include a reservoir to contain the liquid medicine and use electromechanical pumping or metering technology to deliver the liquid medicine via tubing to a needle and/or soft cannula that is inserted subcutaneously into the patient. These existing devices allow control and programming via electromechanical buttons or switches located on the housing of the device. The devices include visual feedback via text or graphic screens and may include alert or warning lights and audio or vibration signals and alarms. Such devices are typically worn in a harness or pocket or strapped to the body of the patient.

Currently available ambulatory infusion devices are expensive, difficult to program and prepare for infusion, and tend to be bulky, heavy and very fragile. Preparing these devices for infusion can be difficult and require the patient to carry both the intended medication as various accessories. Many existing devices also require specialized care, maintenance, and cleaning to assure proper functionality and safety for their intended long-term use. Due to the complexity and high cost of existing devices many patients who would benefit from an ambulatory infusion pump are, nonetheless using inferior forms of therapy.

Accordingly, there is a need for a fluid delivery device with a reduced size and complexity and that is relatively inexpensive to manufacture.

### BRIEF DESCRIPTION OF THE DRAWINGS

These and other features and advantages will be better understood by reading the following detailed description, taken together with the drawings wherein:
FIG. 1 is a top view of a fluid delivery device consistent with one embodiment of the present invention.
FIG. 2 is an exploded view of one embodiment of a fluid driving mechanism used in a fluid delivery device.
FIG. 3 is a perspective view of the fluid driving mechanism shown in FIG. 2 assembled into a chassis.
FIG. 4 is a perspective view of one embodiment of an actuating mechanism for a fluid delivery device.
FIG. 5 is a plan view of the actuating mechanism shown in FIG. 4.actuating the fluid driving mechanism shown in FIG. 2.
FIG. 6 is a schematic diagram of a fill sensor that may be used in a fluid delivery device consistent with one embodiment of the present invention.
FIGS. 7A and 7B are schematic diagrams illustrating the operation of the fill sensor shown in FIG. 6 from a side view.
FIGS. 8A and 8B are schematic diagrams illustrating the operation of a rotational sensor that may be used in a fluid delivery device consistent with one embodiment of the present invention.
FIG. 9 is a perspective view of a chassis of a fluid deliver device with sensors positioned in the chassis consistent with one embodiment of the present invention.
FIG. 10 is a perspective view of a fluid driving mechanism engaging the sensors in the chassis shown in FIG. 9.
FIGS. 11A and 11B are schematic diagrams illustrating the operation of a thread engaging mechanism moving from a non-thread-engaging position to a thread engaging position consistent with one embodiment of the present invention.
FIG. 12A is a perspective views of a thread engaging mechanism in a first position consistent with one embodiment of the present invention.
FIG. 12B is a perspective views of a thread engaging mechanism of FIG. 12A in a second position.
FIG. 12C is a top views of a thread engaging mechanism of FIG. 12A in a second position.
FIG. 13 is an enlarged schematic side view of a drive wheel of a fluid delivery device consistent with one embodiment of the present invention.
FIG. 14 is a schematic diagram of a fill sensor system that may be used in a fluid delivery device consistent with one embodiment of the present invention.
FIGS. 15A, 15B and 15C are schematic diagrams illustrating the operation of an alternative rotational sensor that may be used in a fluid delivery device consistent with one embodiment of the present invention.
FIG. 16 is a bottom view of a fluid delivery device having a needle cap consistent with one embodiment of the present invention.
FIG. 17 is a schematic view of an activation system for a fluid delivery device consistent with one embodiment of the present invention.

### DETAILED DESCRIPTION

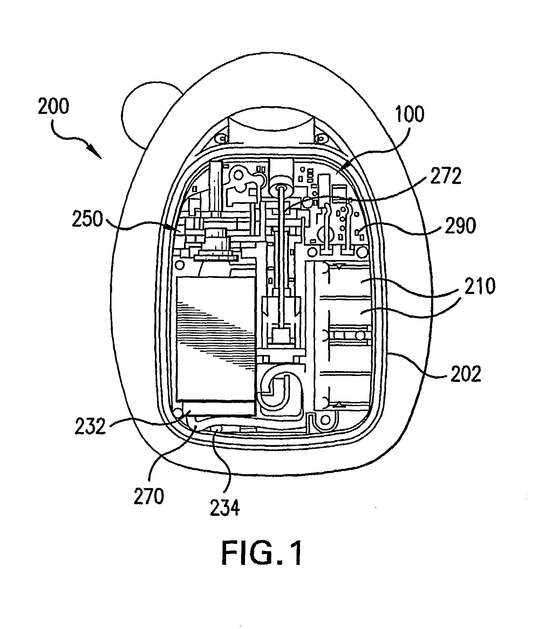

Referring to FIG. 1, one embodiment of a fluid delivery device 200 is shown and described. In the exemplary embodiment, the fluid delivery device 200 is used to subcutaneously deliver a fluid, such as a liquid medicine, to a person or an animal. Those skilled in the art will recognize that the fluid delivery device 200 may be used to deliver other types of fluids. The fluid delivery device 200 may be used to delivery fluids in a controlled manner, for example, according to fluid delivery profiles accomplishing bolus requirements, continuous infusion and variable flow rate delivery.

According to one embodiment, the fluid delivery device 200 may include one or more batteries 210 for providing a power source, a fluid reservoir 230 for holding a fluid, a fluid driving mechanism 250 for driving the fluid out of the reservoir 230, a fluid passage mechanism 270 for receiving the fluid from the reservoir 230, a fluid passage mechanism 270 for receiving the fluid from the reservoir 230 and passing the fluid to a destination, and a circuit board 290 with control circuitry for controlling the device. The fluid delivery device 200 may include a chassis 100 that provides mechanical and/or electrical connections between components of the fluid deliver device 200. One example of the chassis 100 is disclosed in greater detail in U.S. Patent Application Serial No. __________ (Attorney Docket No. INSL-165), which is filed concurrently herewith and incorporated herein by reference. The fluid delivery device 200 may also include a housing 202 to enclose the components 210, 230, 250, 270, 290 and the chassis 100.

One embodiment of the reservoir 230 includes an outlet port 232 for allowing fluid to exit the reservoir 230. The reservoir 230 may also include an inlet port 234 for allowing the reservoir 230 to be filled with fluid. One embodiment of the reservoir 230 is disclosed in greater detail in U.S. Patent Application Serial No. 10/907286, which is filed concurrently herewith and has been incorporated by reference.

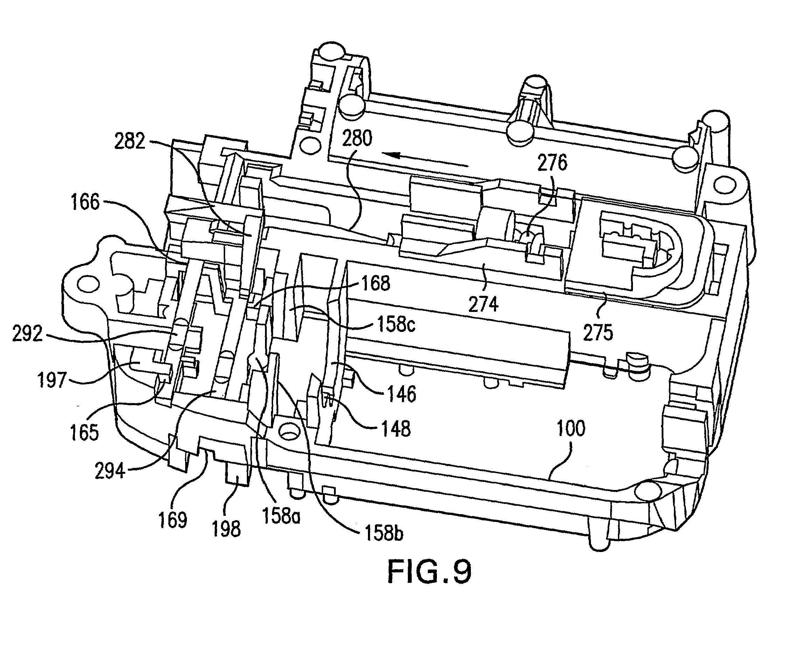

One embodiment of the fluid passage mechanism 270 includes a transcutaneous access tool 272, such as a needle and/or soft cannula, which is capable of penetrating the skin of a patient and passing the fluid into the patient. A fluid path such as tubing (not shown) may be used to fluidly couple the reservoir 230 to the transcutaneous access tool 272. The access tool 272 is mounted to an insertion mechanism, which may include sliding carriages 274, 275, one or more springs 276, and a release member 280 (see FIG. 9). The sliding carriages 274, 275 may be held in a first retracted position until the release member 280 causes the sliding carriages 274, 275 to be released and the spring(s) 276 drive the sliding carriages 274, 275 in the direction of the arrow into an insertion position. The drive mechanism 250 may be used to trigger the release member 280 by engaging an arm 282 of the release member 280. In one embodiment, the sliding carriages 274, 275 first insert a needle and a soft cannula and then the sliding carriage 275 withdraws the needle leaving the soft cannula in place. Various transcutaneous access devices and systems that may be accommodated in a chassis in accordance with the present invention are disclosed in, for example, U.S. Patent No. 6,656,159 and U.S. Patent Application Ser. Nos. 10/128,206, 10/195,745, 10/260,192, 10/261,003, all of which are hereby incorporated by reference.

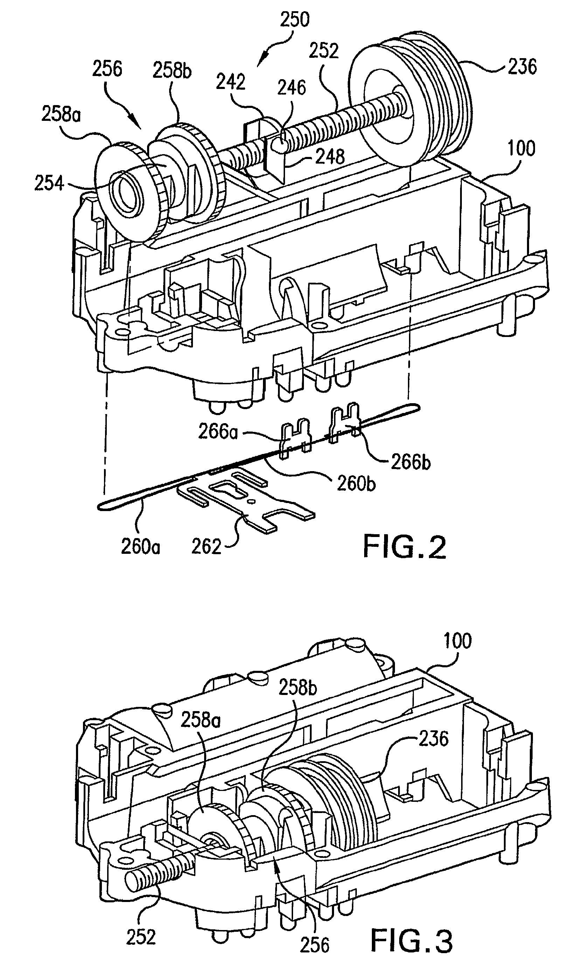

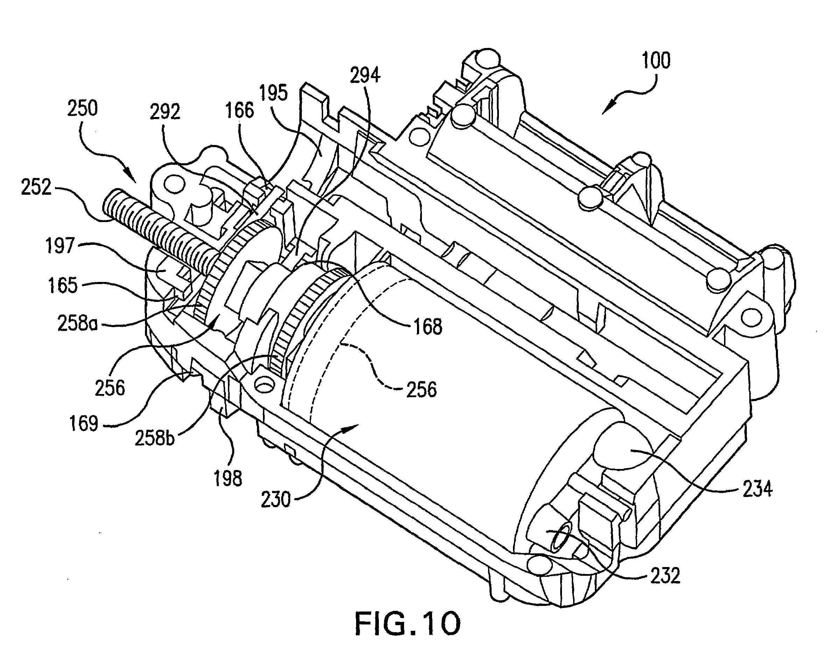

Referring to FIGS. 2 and 3, one embodiment of the fluid driving mechanism 250 includes a threaded drive rod 252 coupled to a plunger 236. The plunger 236 is received in the fluid reservoir 230 (see FIG. 10). In the illustrated embodiment, the drive wheel 256 threadably engages and imparts linear motion to the threaded drive rod 252 to advance the plunger 236 into the reservoir 230, thereby forcing fluid out of the reservoir 230. Alternatively, the threaded drive rod 252 may threadably engage the plunger 236 and the drive wheel 256 may be fixed to the drive rod 252 to rotate the drive rod 252, which imparts linear motion to the plunger 236.

One embodiment of the drive wheel 256 includes first and second ratchet wheel portions 258a, 258b and a hub 254 between the ratchet wheel portions 258a, 258b. The drive wheel 256 may be rotatably supported by the chassis 100. In particular, the hub 254 may be supported by one or more bearing surfaces 158a-c on the chassis 100 (see FIG. 9). The drive wheel 256 is preferably be made of plastic and the drive rod 252 is preferably made of metal or plastic.

An actuation mechanism is used to engage and incrementally rotate the drive wheel 256. One embodiment of an actuating mechanism for the drive wheel 256 may include a linear actuator. A preferred linear actuator comprises a shape memory allow wire. As shown in FIGS. 2 and 3, preferably, the SMA wire comprises first and second shape memory alloy (SMA) wire portions 260a, 260b coupled to a pivotable drive engaging member 262. The SMA wire portions 260a, 260b may be formed as a continuous SMA wire crimped to the pivotable drive engaging member 262 as shown. The SMA wire portions 260a, 260b may also be formed as separate pieces of wire connected to the pivotable drive engaging member 262 and may be connected in other ways known to those skilled in the art. The SMA wire may be made of nitinol, a well known alloy of nickel and titanium, or other SMA wire known to those skilled in the art. The pivotable drive engaging member 262 may be made of an electrically conductive material such as copper alloy.

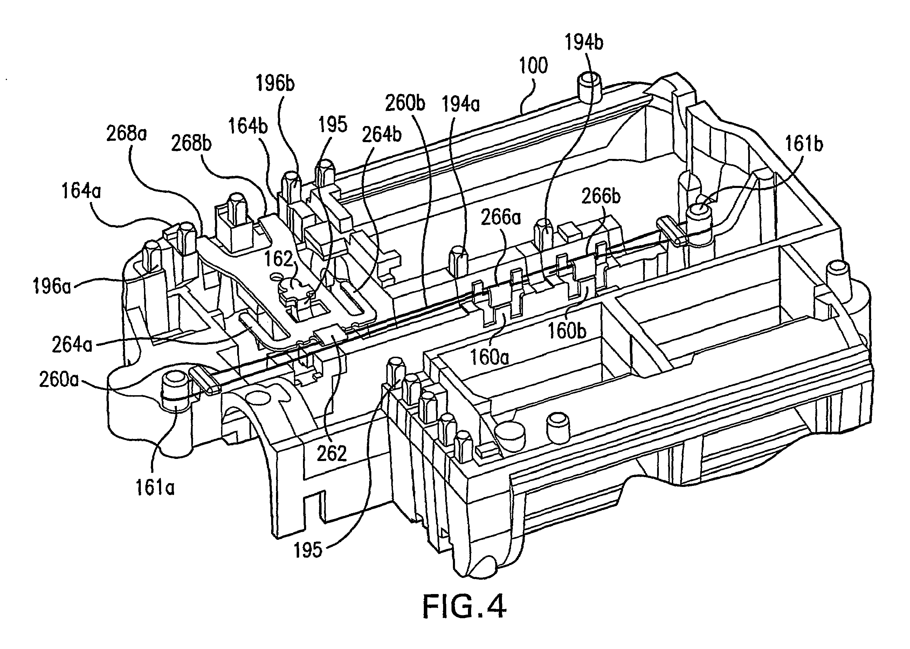

As shown in FIG. 4, the SMA wire portions 260a, 260b may be mechanically secured to the chassis 100 and electrically connected to contact points 160a, 160b on the chassis 100. In the illustrated embodiment, the SMA wire portions 260a, 260b are connected to crimp terminations 266a, 266b which are mounted in slots formed in an electrically conductive portion of the chassis 100. The crimp terminations 266a, 266b may be made of a conductive material such as copper alloy. The SMA wire portions 260a, 260b may also wrap around posts 161a, 161b or other mechanical structures, for example, extending from the chassis 100 to increase the functional length of the SMA wire.

The pivotable drive engaging member 262 may be pivotably coupled to a pivot point 162 on the chassis 100, which also acts as an electrical contact point. One embodiment of the pivotable drive engaging member 262 includes arms 264a, 264b that engage teeth on the ratchet wheel portions 258a, 258b and incrementally rotating the drive wheel 256 when the pivotable drive engaging member 262 pivots. The pivotable drive engaging member 262 may also include legs 268a, 268b that contact points 164a, 164b on the chassis 100 when the pivotable drive engaging member 262 pivots. Alternatively, the pivotable drive engaging member 262 may only include one arm and one leg.

The contact points 160a, 160b, 162, 164a, 164b provide an electrical connection between the components of the actuating mechanism and the control circuitry (not shown). In one embodiment, this electrical connection is provided via conductive paths extending along the chassis 100. The contact points 160a, 160b electrically connect the SMA wire portions 260a, 260b to actuator conductive paths 194a, 194b on the chassis 100. The pivot point 162 electrically connects the pivotable drive engaging member 262 to a common ground conductive path 195 on the chassis 100. The contact points 164a, 164b electrically connect the pivotable drive engaging member 262 to actuator conductive paths 196a, 196b. The conductive paths along the chassis 100 are shown and described in greater detail in U.S. Patent Application Serial No: 10/907286, which is filed concurrently herewith and has been incorporated by reference. Those skilled in the art will recognize other means for electrically connecting the components of the actuating mechanism to the control circuitry.

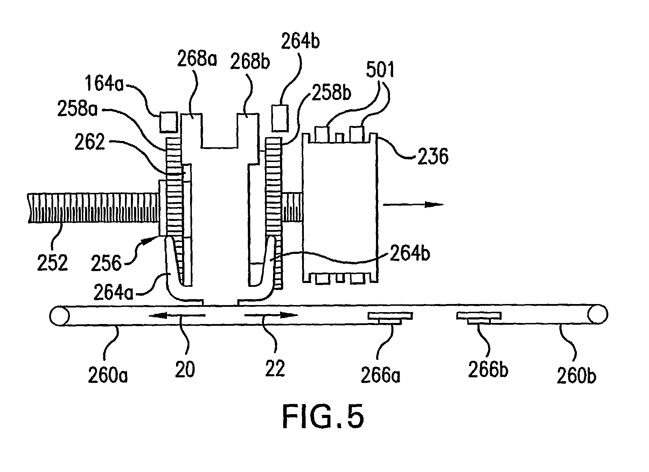

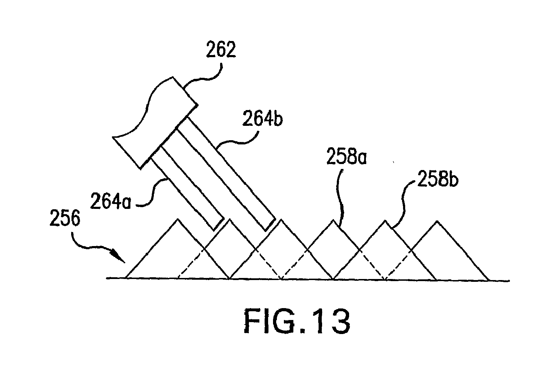

Referring to FIG. 5, the operation of the actuation mechanism is described in greater detail. To charge the SMA wire portions 260a, 260b, control circuitry (not shown) electrically connected to the chassis 100 may selectively apply current to the SMA wire portions 260a, 260b, for example, via the conductive paths 194a, 194b (shown in FIG. 4). Current applied to a first SMA wire portion 260a passes through the SMA wire portion 260a and through the pivotable drive engaging member 262 to the common ground conductive path 195 via the pivot point 162 (shown in FIG. 4), thereby energizing the first SMA wire portion 260a without energizing the second SMA wire portion 260b. When charged, the first SMA wire portion 260a contracts and pulls the pivotable drive engaging member 262 in a first direction indicated by arrow 20. When the pivotable drive engaging member 262 pivots in the first direction 20, the arm 264a engages a tooth on the ratchet wheel portion 258a causing the drive wheel 256 to rotate one increment. The pivotable drive engaging member 262 pivots in the first direction 20 until the leg 268b contacts the contact point 164b, electrically connecting the pivotable drive engaging member 262 to the conductive path 196b (shown in FIG. 4). This activates a signal to the control circuitry (not shown) via the conductive path 196b indicating that the control circuitry should disable the current being applied to the first SMA wire portion 260a. At all times one of the arms 264a or 264b is engaged by the tooth portions of the drive wheel. The engaged arm 264a or 264b, therefore prevents reverse rotation of the drive eliminating the need for a separate pawl element. As shown in FIG 13, the teeth of the wheel portions 258a and 258b are offset relative to one another thereby maximizing the tolerances for the interface between the arms 264a and 264b and wheels 258a and 258b.

To initiate another pulse, the control circuitry applies current to the second SMA wire portion 260b, for example, via the conductive path 194b and the contact point 160b (shown in FIG. 4). When charged, the second SMA wire portion 260b contracts and pulls the pivotable drive engaging member 262 in a second direction indicated by arrow 22. When the pivotable drive engaging member 262 pivots in the second direction 22, the arm 264b engages a tooth on the ratchet wheel portion 258b causing the drive wheel 256 to rotate one increment. The pivotable drive engaging member 262 pivots in the second direction 22 until the leg 268a contacts the contact point 164a, electrically connecting the pivotable drive engaging member 262 to the conductive path 196a (shown in FIG. 4). This activates a signal to the control circuitry (not shown) via the conductive path 196a indicating that the control circuitry should disable the current being applied to the second SMA wire portion 260b.

Each incremental rotation of the drive wheel 256 advances the plunger in the reservoir 230 to cause a discrete amount of fluid to be dispensed. The discrete amount of fluid to be dispensed is a function of the lead screw pitch (i.e., threads/inch), the number of teeth on the ratchet wheel (that is, ratchet wheel tooth size) and the diameter of the fluid reservoir. In a preferred embodiment, for delivering U100 insulin for treatment of Type I diabetes, the discrete amount of fluid to be dispensed is between about 0.25 µL and about 0.5 µL. The control circuitry alternates energizing the SMA wire portions 260a, 260b until a desired amount of fluid has been dispensed. One example of the control circuitry and control method is disclosed in greater detail in U.S. Patent App. Ser. Nos. 10/835727, 10/836525, and 10/836535, which are fully incorporated herein by reference.

Examples of alternative fluid driving mechanisms and actuation mechanisms that may be used in the fluid delivery device 200 are disclosed in U.S. Patent Nos. 6,656,158 and 6,656,159 and U.S. Patent Application Serial No. 10/704,291, all of which are incorporated herein by reference.

The fluid delivery device 200 may also include one or more sensors to monitor operation of the fluid driving mechanism 250. For example, fill sensors may be used to sense a level of fluid in the reservoir 230 when filling the reservoir 230 with fluid and/or dispensing fluid from the reservoir 230. Rotational sensors may be used to sense that the driving mechanism 250 is operating in accordance with expectations.

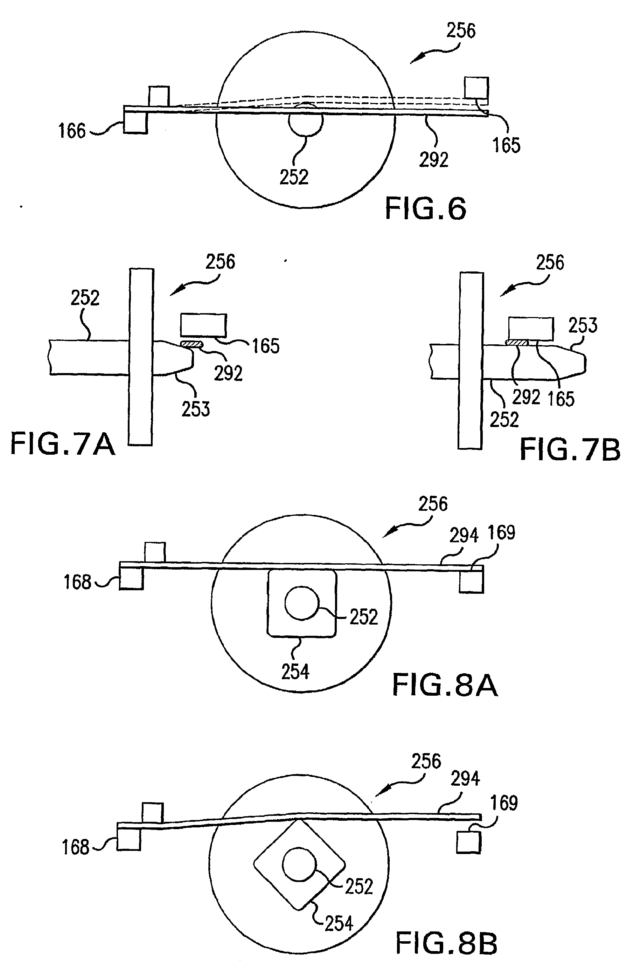

Referring to FIGS. 6, 7A and 7B, one embodiment of a fill sensor includes a sensor bar 292 supported or mounted at one end by a sensor support 166 and extending across the path of the threaded drive rod 252. When the threaded drive rod 252 engages the fill sensor bar 292, the drive rod 252 moves the fill sensor bar 292 and causes it to engage a contact point 165. Engaging the contact point 165 completes a circuit and activates a fill sensor signal to the control circuitry. The fill sensor bar 292 may be made of an electrically conductive material such as a copper alloy and/or may be plated with a conductive material such as nickel or gold.

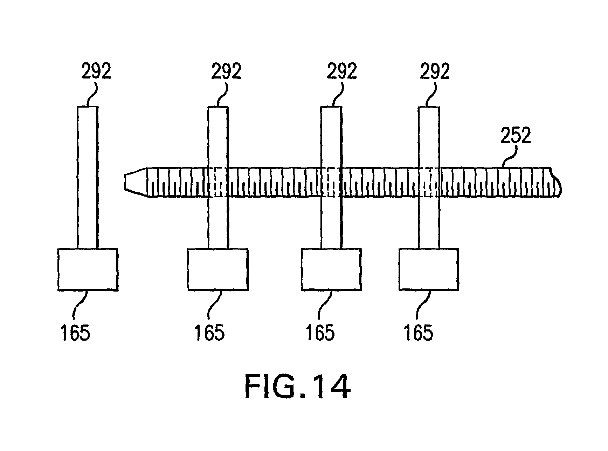

At the point when the drive rod 252 causes the fill sensor bar 292 to engage the contact point 165, the plunger 236 is at a predetermined location in the reservoir 230 and the amount of fluid in the reservoir 230 is known. The fill sensor may be used when filling the fluid delivery device 200 to indicate that the reservoir has been filled to a predetermined amount and/or to "wake up" the control circuitry in the fluid delivery device 200. Thus, in a preferred embodiment of the present invention, before the fill sensor has been triggered, the fluid delivery device control circuitry is in a rest state characterized by minimal activity and power consumption. In such an embodiment, once the fill sensor is triggered, the control circuitry enters an active state characterized by higher activity and power consumption. The fill sensor may also be used when delivering the fluid to indicate that the predetermined amount of fluid remains in the reservoir. Although one embodiment of a fill sensor is shown and described, other configurations may be used to activate a fill sensor signal in response to a predetermined position of the drive rod. Referring to FIG. 14, in an alternative embodiment, the fill sensing system comprises a plurality of sensor bars 292 and contact points 165 placed at desired positions along the drive rod 252. The number and position of sensor bars and contact points may be selected for any desired resolution.

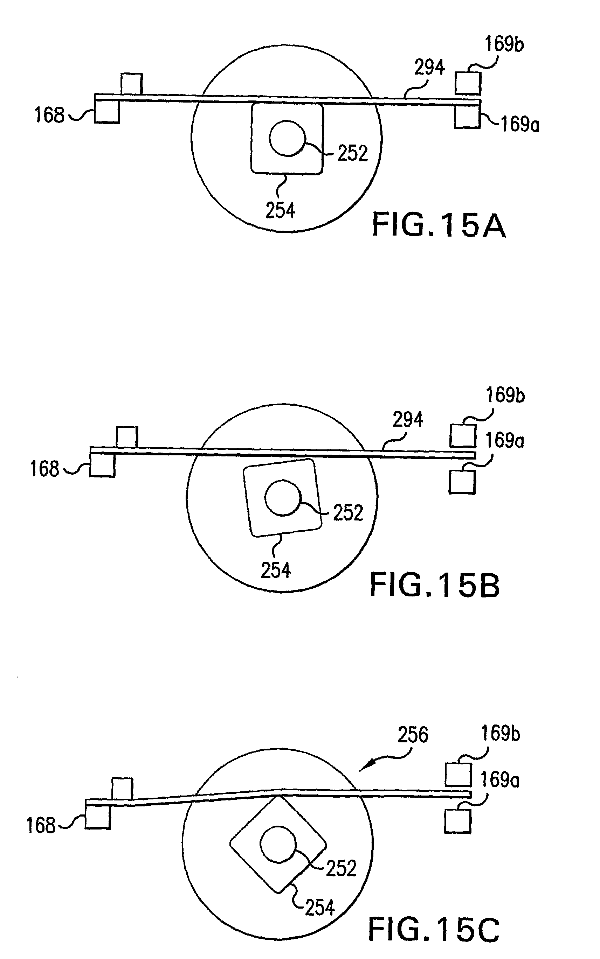

Referring to FIGS. 8A and 8B, one embodiment of a rotational sensor includes a rotational sensor bar 294 supported or mounted at one end by a sensor support 168 and extending across a polygonal (or other camming) shaped portion of the drive wheel 256. The rotational sensor bar 294 contacts a sensor contact point 169 to complete a circuit and activate a rotational sensor signal to the control circuitry. When the drive wheel 256 incrementally rotates, the hub 254 of the drive wheel 256 engages the rotational sensor bar 294 and moves the rotational sensor bar 294 out of contact with the contact point 169. The activation and deactivation of the rotational sensor signal indicates that the drive wheel 256 is rotating. The specific shape of the polygonal portion of the drive wheel may be selected to optimize the resolution of the rotational sensor for a given application. In an alternative embodiment, as shown in FIGS. 15A, 15B and 15C, a rotational sensor may comprise a sensor bar positioned to alternate between two contact points 169a and 169b, thereby having 3 different states as shown in FIGS. 15A, 15B and 15C, respectively, and a corresponding increase in resolution. Although one embodiment of a rotational sensor is shown and described, other configurations may be used to activate a rotational sensor signal in response to movement of the drive wheel.

As shown in FIGS. 9 and 10, the sensor bars 292, 294 may be supported or mounted in the chassis 100. In this embodiment, the sensor supports 166, 168 are electrically connected to the common ground conductive path 195 extending along the chassis 100 and the contact points 165, 169 are electrically connected to sensor conductive paths 197, 198, respectively, extending along the chassis 100. The sensor conductive paths 197, 198 may be electrically connected to the control circuitry.

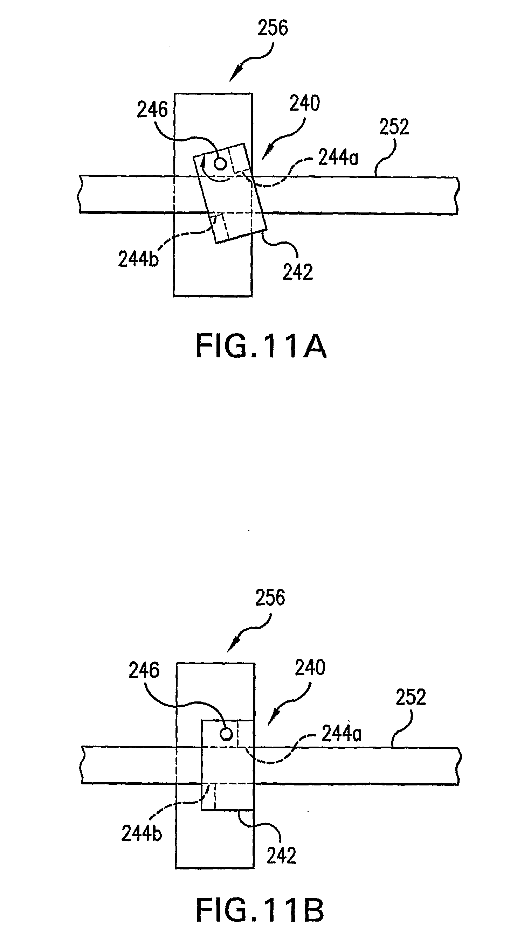

Referring to FIGS. 11A and 11B, the drive wheel 256 may include a thread engaging mechanism 240 that moves from a non-thread-engaging position to a thread engaging position. The non-thread-engaging position allows the threaded drive rod 252 to pass freely through the drive wheel 256, for example, when the reservoir of the fluid delivery device is being filled. The thread engaging position allows the threaded drive rod 252 to be advanced when the drive wheel 256 is rotated, for example, when dispensing or delivering fluid from the reservoir.

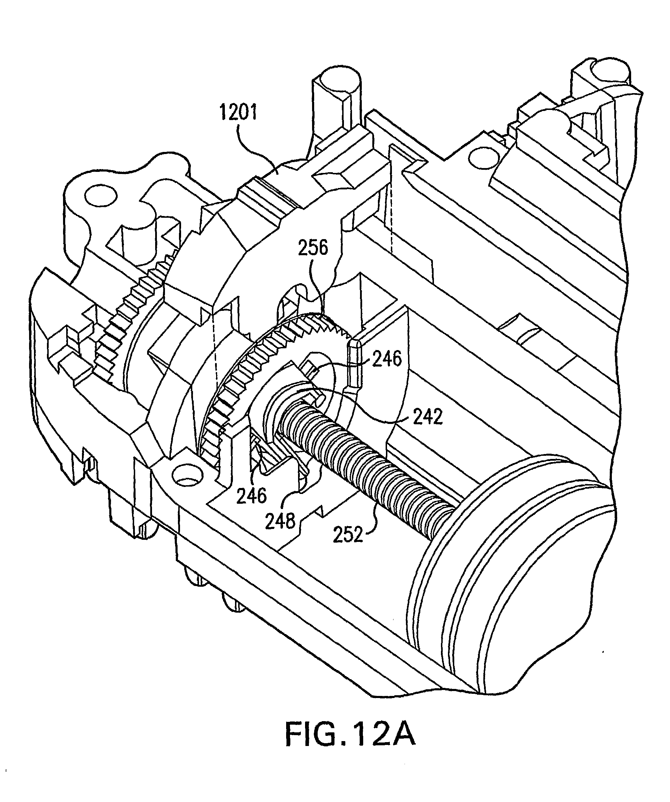

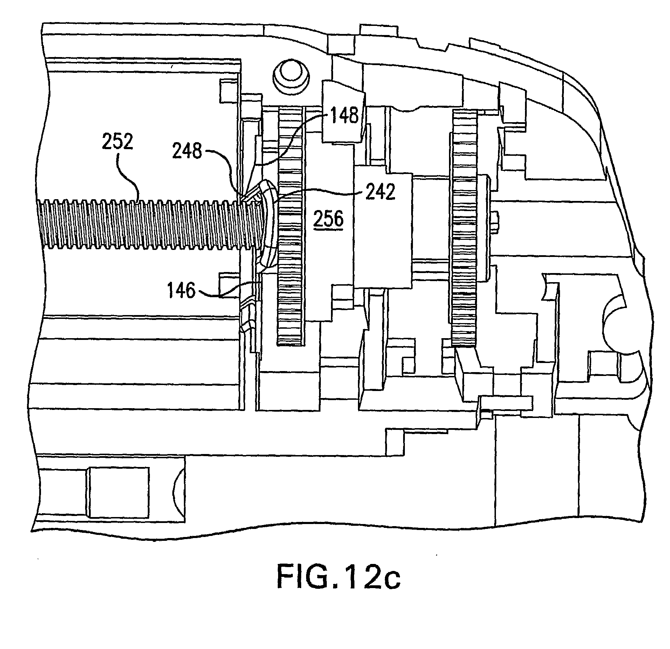

One embodiment of the thread engaging mechanism 240 includes a tilt nut 242 having threaded regions 244a, 244b. The tilt nut 242 may include one or more pivot pins 246 that pivotably support the tilt nut 242 within the drive wheel 256 (see FIG. 2). The tilt nut pins 246 provide a pivot axis for the tilt nut 242 that is perpendicular to that of the drive rod 252. The pivot axis is preferably offset from the center of the tilt nut 242. The tilt nut 242 is retained in the drive wheel 256 by portions of the chassis and by the tilt nut clip 1201, which is shown exploded in FIG. 12A. When the tilt nut 242 is in the open (tilted) position, the threaded regions 244a, 244b do not engage the threads on the threaded drive rod 252 (FIG. 11A). When the tilt nut 242 is in the closed position (FIG. 11B) the threaded portions engage the threaded drive rod 252.

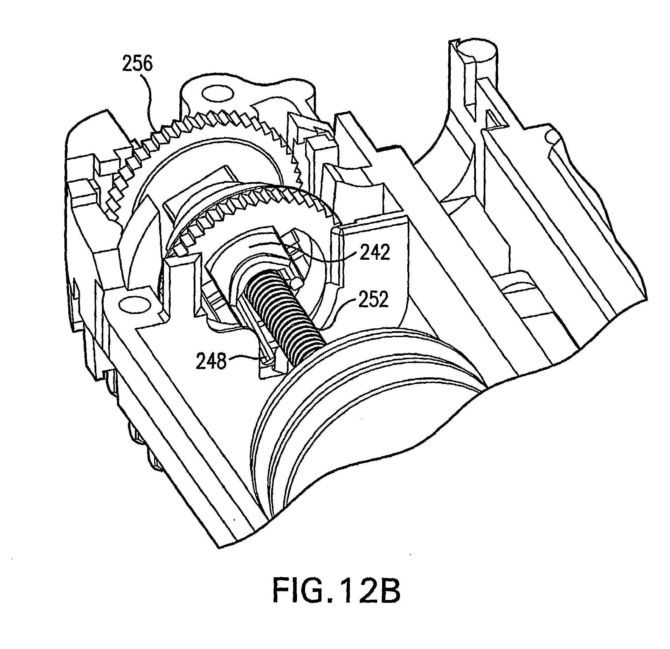

According to one embodiment, shown in FIGS. 12A and 12B, the tilt nut 242 may also include a cam member 248 that engages guide surface 146 and camming surface 148 on the chassis 100. Initially, the cam member 248 engages the guide surface 146 and is maintained in the open (tilted) position (FIG. 12A). During initial rotation, the cam member 248 engages the camming surface 148 and the camming surface 148 forces the cam member 248 to pivot into the thread engaging position. The tilt nut 242 may remain in the thread engaging position as the drive wheel 256 rotates to advance the drive rod 252 and the plunger 236, thereby dispensing fluid. Once the drive rod 252 is engaged by the tilt nut 252, the tilt nut is retained in the closed position by the surfaces of the chassis and the tilt nut clip and by the pressure of the drive rod against the offset pivot axis of the tilt nut 242 in the direction of the plunger 236. Notably, in a preferred embodiment, the stiction of the o-rings 501 on the plunger 236 provides sufficient anti-rotational friction to prevent the plunger from rotating with the drive rod 252.

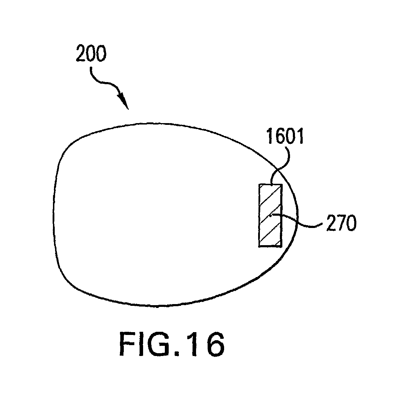

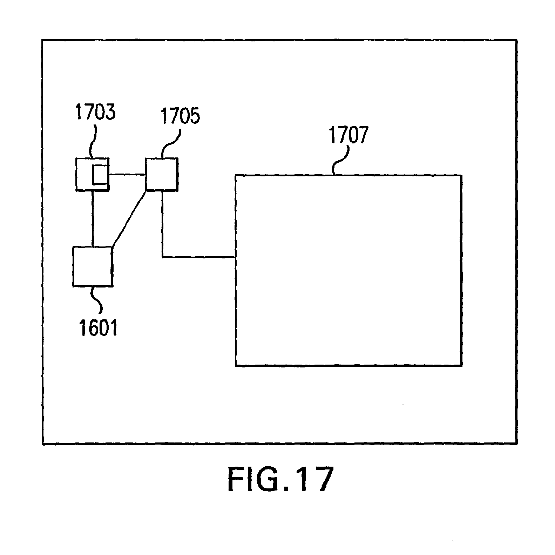

FIGS. 16 and 17 show an alternative embodiment of a "wake-up" system for a fluid delivery device. FIG. 16 shows the bottom surface of a fluid delivery device having a transcutaneous access tool 270 and a needle cap 1601. As shown in FIG. 17, the needle cap is electrically and/or mechanically connected to a switch 1705 that couples a power source 1703 and the control circuitry 1705. Removal of the cap 1601 closes the switch between the power source 1703 and the control circuitry 1705 thereby "waking" the fluid delivery device.

Although the illustrated embodiment shows the components mechanically secured to and electrically connected to a single chassis, the components of the fluid delivery device consistent with embodiments of the present invention may be mechanically secured and/or electrically connected to other structures such as separate structural members or the housing.

In summary, a fluid delivery device, consistent with one embodiment of the present invention, includes a fluid reservoir, a plunger received in the fluid reservoir, a threaded drive rod coupled to the plunger to advance the plunger in the fluid reservoir, and a drive wheel coupled to the threaded drive rod. The fluid delivery device also includes a pivotable drive engaging member including at least one arm configured to engage and incrementally rotate the drive wheel and at least first and second shape memory alloy (SMA) wire portions coupled to the pivotable drive engaging member to pivot the pivotable drive engaging member. In response to being charged, the first SMA wire portion is configured to contract and pull the pivotable drive engaging member in a first direction and the second SMA wire portion is configured to contract and pull the pivotable drive engaging member in a second direction.

Consistent with another embodiment of the present invention, a fluid delivery device includes a fluid reservoir, a plunger received in the fluid reservoir, a threaded drive rod coupled to the plunger to advance the plunger in the fluid reservoir, a drive wheel coupled to the threaded drive rod, and an actuating mechanism configured to engage and incrementally rotate the drive wheel. This embodiment of the fluid delivery device also includes a rotational sensor extending across the drive wheel. The drive wheel is configured to engage the rotational sensor during rotation to cause the rotational sensor to contact a conductive path activating an electrical signal indicating rotation of the drive wheel.

Consistent with a further embodiment of the present invention, a fluid delivery device includes a fluid reservoir, a plunger received in the fluid reservoir, a threaded drive rod coupled to the plunger to advance the plunger in the fluid reservoir, a drive wheel configured to threadably engage the threaded drive rod to impart linear motion to the threaded drive rod when the drive wheel rotates, and an actuating mechanism configured to engage and incrementally rotate the drive wheel. This embodiment of the fluid delivery device also includes a fill sensor extending across a path of the threaded drive rod. The threaded drive rod is configured to engage the fill sensor when the plunger is at a predetermined location in the fluid reservoir to cause the fill sensor to contact an electrically conductive path enabling an electrical signal indicating an amount of fluid in the fluid reservoir.

Consistent with yet another embodiment, a fluid delivery device includes a fluid reservoir, a plunger received in the fluid reservoir, and a threaded drive rod coupled to the plunger to advance the plunger in the fluid reservoir. In this embodiment, the fluid delivery device also includes a drive wheel configured to receive the threaded drive rod and including a thread engaging mechanism configured to move from a non-thread-engaging position to a thread engaging position in which the thread engaging mechanism engages threads of the threaded drive rod. The drive wheel is configured to impart linear motion to the threaded drive rod when the thread engaging mechanism is in the thread engaging position and the drive wheel rotates.

While the principles of the invention have been described herein, it is to be understood by those skilled in the art that this description is made only by way of example and not as a limitation as to the scope of the invention. Other embodiments are contemplated within the scope of the present invention in addition to the exemplary embodiments shown and described herein. Modifications and substitutions by one of ordinary skill in the art are considered to be within the scope of the present invention, which is not to be limited except by the following claims.

### CLAUSES

The invention can be further defined with respect to the following consistory clauses:
1. A fluid delivery device comprising:
   a fluid reservoir;
   a plunger received in said fluid reservoir;
   a threaded drive rod coupled to said plunger to advance said plunger in said fluid reservoir; a drive wheel coupled to said threaded drive rod;
   a pivotable drive engaging member including at least one arm configured to engage and incrementally rotate said drive wheel; and
   a linear actuator coupled to said pivotable drive engaging member to pivot said pivotable drive engaging member, wherein said linear actuator causes the pivotable drive engaging member to pivot when actuated.
2. The fluid delivery device of clause 1, wherein the linear actuator is a SMA wire.
3. The fluid delivery device of clause 2, wherein the SMA wire comprises at least first and second SMA wire portions wherein activation of the first SMA wire portion causes the pivotable drive engaging member to pivot in a first direction and wherein activation of the second SMA wire portion causes the pivotable drive engaging member to pivot in a second direction.
4. The fluid delivery device of clause 3 wherein said first and second SMA wire portions are part of a continuous SMA wire having first and second ends, wherein said pivotable drive engaging member is coupled between said first and second ends.
5. The fluid delivery device of clause 1 wherein said pivotable drive engaging member includes at least one leg configured to contact an electrically conductive path to activate an actuator signal.
6. The fluid delivery device of clause 5 wherein said pivotable drive engaging member includes: first and second arms configured to engage and incrementally rotate said drive wheel as said drive engaging member pivots in respective said first and second directions.
7. The device of clause 6, wherein the pivotable drive engaging member comprises first and second legs configured to contact electrically conductive paths to activate actuator signals as said drive engaging member pivots in respective said first and second directions.
8. The fluid delivery device of clause 6 wherein said drive wheel includes first and second ratchet wheel portions configured to be engaged by said first and second arms of said pivotable drive engaging member.
9. The fluid delivery device of clause 6, wherein the first and second ratchet wheel portions comprise a plurality of teeth for engaging the arms of the pivotable drive engaging member and wherein the teeth of the first ratchet wheel are offset relative to the teeth of the second ratchet wheel.
10. The fluid delivery device of clause 3 further comprising a chassis mechanically interfacing said fluid reservoir, said drive wheel, said pivotable drive engaging member and said SMA wire portions.
11. The fluid delivery device of clause 10 wherein said pivotable drive engaging member is pivotably coupled to said chassis, and wherein said SMA wire is mounted to said chassis at said first and second ends.
12. The fluid delivery device of clause 11 wherein said chassis includes electrically conductive paths, wherein said first and second SMA wire portions are electrically connected to two of said conductive paths configured to conduct electrical current to said SMA wire portions, and wherein said pivotable drive engaging member is electrically connected to another one of said conductive paths configured to act as a common ground.
13. The fluid delivery device of clause 1 further comprising a rotational sensor extending across said drive wheel, said drive wheel being configured to engage said rotational sensor during at least a portion of rotation of said drive wheel to cause said rotational sensor to engage and disengage an electrically conductive path thereby activating a rotational sensor signal indicating rotation of said drive wheel.
14. The fluid delivery device of clause 13 further comprising a chassis mechanically interfacing said fluid reservoir, said drive wheel, said pivotable drive engaging member and said SMA wire portions, wherein said chassis includes electrically conductive paths, wherein one end of said rotational sensor is mounted to said chassis and electrically connected to one of said conductive paths, and wherein said rotational sensor is configured to move into and out of contact with another of said electrically conductive paths to activate said rotational sensor signal.
15. The fluid delivery device of clause 1 wherein said drive wheel is configured to threadably engage said threaded drive rod to impart linear motion to said threaded drive rod.
16. The fluid delivery device of clause 15 further comprising a fill sensor extending across a path of said threaded drive rod, said threaded drive rod being configured to engage said fill sensor when said plunger is at a predetermined location in said fluid reservoir to cause said fill sensor to contact a conductive path activating a fill sensor signal indicating an amount of fluid in said fluid reservoir.
17. The fluid delivery device of clause 16 further comprising a chassis mechanically interfacing said fluid reservoir, said drive wheel, said pivotable drive engaging member and said SMA wire portions, wherein said chassis includes electrically conductive paths, wherein one end of said fill sensor is mounted to said chassis and electrically connected to one of said conductive paths, and wherein said fill sensor is configured to move into contact another of said conductive paths to activate said fill sensor signal.
18. The fluid delivery device of clause 15 comprising a plurality of fill sensors extending across a path of said threaded drive rod, said threaded drive rod being configured to engage said fill sensors when said plunger is at predetermined locations in said fluid reservoir to cause said fill sensors to contact a conductive path activating fill sensor signals, each indicating an amount of fluid in said fluid reservoir.
19. The fluid delivery device of clause 1 wherein said drive wheel includes a thread engaging mechanism configured to move from a non-thread-engaging position to a thread engaging position in which said thread engaging mechanism engages threads of said threaded drive rod, said drive wheel being configured to impart linear motion to said threaded drive rod when said thread engaging mechanism is in said thread engaging position and said drive wheel rotates.
20. A fluid delivery device comprising: a fluid reservoir; a plunger received in said fluid reservoir; a threaded drive rod coupled to said plunger to advance said plunger in said fluid reservoir; a drive wheel coupled to said threaded drive rod; an actuating mechanism configured to engage and incrementally rotate said drive wheel; and a rotational sensor extending across said drive wheel, said drive wheel being configured to engage said rotational sensor during rotation to cause said rotational sensor to contact a conductive path activating an electrical signal indicating rotation of said drive wheel.
21. The fluid delivery device of clause 20 further comprising a chassis mechanically interfacing said fluid reservoir, said drive wheel, and said actuating mechanism, wherein said chassis includes electrically conductive paths, wherein one end of said rotational sensor is mounted to said chassis and electrically connected to one of said conductive paths, and wherein said rotational sensor is configured to move into and out of contact with another of said electrically conductive paths to activate said electrical signal.
22. The fluid delivery device of clause 20 wherein said drive wheel includes a hub configured to contact said rotational sensor.
23. A fluid delivery device comprising: a fluid reservoir; a plunger received in said fluid reservoir; a threaded drive rod coupled to said plunger to advance said plunger in said fluid reservoir; a drive wheel configured to threadably engage said threaded drive rod, said drive wheel being configured to impart linear motion to said threaded drive rod when said drive wheel rotates; an actuating mechanism configured to engage and incrementally rotate said drive wheel; and a fill sensor extending across a path of said threaded drive rod, said threaded drive rod being configured to engage said fill sensor when said plunger is at a predetermined location in said fluid reservoir to cause said fill sensor to contact an electrically conductive path enabling an electrical signal indicating an amount of fluid in said fluid reservoir.
24. The fluid delivery device of clause 23 comprising a plurality of fill sensors extending across a path of said threaded drive rod, said threaded drive rod being configured to engage said fill sensors when said plunger is at predetermined locations in said fluid reservoir to cause said fill sensors to contact a conductive path activating fill sensor signals, each indicating an amount of fluid in said fluid reservoir.
25. The fluid delivery device of clause 23 further comprising a chassis mechanically interfacing said fluid reservoir, said drive wheel, and said actuating mechanism, wherein said chassis includes electrically conductive paths, wherein one end of said fill sensor is mounted to said chassis and electrically connected to one of said conductive paths, and wherein said fill sensor is configured to move into contact with another of said electrically conductive paths to activate said electrical signal.
26. A fluid delivery device comprising: a fluid reservoir; a plunger received in said fluid reservoir; a threaded drive rod coupled to said plunger to advance said plunger in said fluid reservoir; a drive wheel configured to receive said threaded drive rod, said drive wheel including a thread engaging mechanism configured to move from a non-thread-engaging position to a thread engaging position in which said thread engaging mechanism engages threads of said threaded drive rod, said drive wheel being configured to impart linear motion to said threaded drive rod when said thread engaging mechanism is in said thread engaging position and said drive wheel rotates; and an actuating mechanism configured to engage and incrementally rotate said drive wheel.
27. The fluid delivery device of clause 26 further comprising a chassis mechanically interfacing said fluid reservoir, said drive wheel, and said actuating mechanism, wherein said chassis includes a camming surface configured to engage said thread engaging mechanism to move said thread engaging mechanism from said non-thread-engaging position to said thread engaging position.

## Claims

1. A fluid delivery device comprising:
a fluid reservoir;
a plunger received in said fluid reservoir;
a threaded drive rod coupled to said plunger to advance said plunger in said fluid reservoir; a drive wheel coupled to said threaded drive rod;
an actuating mechanism configured to engage and incrementally rotate said drive wheel; and
a rotational sensor extending across said drive wheel, said drive wheel being configured to engage said rotational sensor during rotation to cause said rotational sensor to contact a conductive path activating an electrical signal indicating rotation of said drive wheel.

2. The fluid delivery device of claim 1 further comprising a chassis mechanically interfacing said fluid reservoir, said drive wheel, and said actuating mechanism, wherein said chassis includes electrically conductive paths, wherein one end of said rotational sensor is mounted to said chassis and electrically connected to one of said conductive paths, and wherein said rotational sensor is configured to move into and out of contact with another of said electrically conductive paths to activate said electrical signal.

3. The fluid delivery device of claim 1 wherein said drive wheel includes a hub configured to contact said rotational sensor.

4. A fluid delivery device comprising:
a fluid reservoir;
a plunger received in said fluid reservoir;
a threaded drive rod coupled to said plunger to advance said plunger in said fluid reservoir;
a drive wheel configured to threadably engage said threaded drive rod, said drive wheel being configured to impart linear motion to said threaded drive rod when said drive wheel rotates;
an actuating mechanism configured to engage and incrementally rotate said drive wheel; and
a fill sensor extending across a path of said threaded drive rod, said threaded drive rod being configured to engage said fill sensor when said plunger is at a predetermined location in said fluid reservoir to cause said fill sensor to contact an electrically conductive path enabling an electrical signal indicating an amount of fluid in said fluid reservoir.

5. The fluid delivery device of claim 4 comprising a plurality of fill sensors extending across a path of said threaded drive rod, said threaded drive rod being configured to engage said fill sensors when said plunger is at predetermined locations in said fluid reservoir to cause said fill sensors to contact a conductive path activating fill sensor signals, each indicating an amount of fluid in said fluid reservoir.

6. The fluid delivery device of claim 4 further comprising a chassis mechanically interfacing said fluid reservoir, said drive wheel, and said actuating mechanism, wherein said chassis includes electrically conductive paths, wherein one end of said fill sensor is mounted to said chassis and electrically connected to one of said conductive paths, and wherein said fill sensor is configured to move into contact with another of said electrically conductive paths to activate said electrical signal.

7. A fluid delivery device comprising:
a fluid reservoir;
a plunger received in said fluid reservoir;
a threaded drive rod coupled to said plunger to advance said plunger in said fluid reservoir;
a drive wheel configured to receive said threaded drive rod, said drive wheel including a thread engaging mechanism configured to move from a non-thread engaging position to a thread engaging position in which said thread engaging mechanism engages threads of said threaded drive rod, said drive wheel being configured to impart linear motion to said threaded drive rod when said thread engaging mechanism is in said thread engaging position and said drive wheel rotates; and
an actuating mechanism configured to engage and incrementally rotate said drive wheel.

8. The fluid delivery device of claim 7 further comprising a chassis mechanically interfacing said fluid reservoir, said drive wheel, and said actuating mechanism, wherein said chassis includes a camming surface configured to engage said thread engaging mechanism to move said thread engaging mechanism from said non-thread-engaging position to said thread engaging position.
